# EUROPEAN PATENT SPECIFICATION

(11) **EP 1 373 180 B1**
(45) Date of publication and mention of the grant of the patent: **17.05.2006**
(21) Application number: 01934292.2
(22) Date of filing: 30.03.2001
(51) Int. Cl.: C07C 46/04, C07C 50/10

(54) **PROCESS FOR PREPARATION OF 2-METHYL-1,4-NAPHTHOQUINONE**
VERFAHREN ZUR HERSTELLUNG VON 2-METHYL-1,4-NAPHTOCHINON
PREPARATION DE 2-METHYL-1,4-NAPHTHOQUINONE

(43) Date of publication of application: 02.01.2004
(73) Proprietor: Council of Scientific and Industrial Research, New Delhi 110 001, Maharashtra (IN)
(72) Inventor: SANKARASUBBIER, N., Indian Inst. of Chemical Tech., Andhra Pradesh (IN); MURTHY, K., c/o Indian Inst. of Chemical Techn., Andhra Pradesh (IN); REDDY, K. M., c/o Indian Inst. of Chemical Techn., Andhra Pradesh (IN); NANDHIKONDA, P., c/o Indian Inst. of Chemical Tech, Andhra Pradesh (IN)
(74) Representative: Manaton, Ross Timothy
(86) International application number: PCT/IN2001/000062
(87) International publication number: WO 2002/079133

(56) References cited:
- US-A- 2 373 003
- CHEMICAL ABSTRACTS, vol. 88, no. 5, 30 January 1978 (1978-01-30) Columbus, Ohio, US; abstract no. 37500, TAKANOBU ETSUYA ET AL: "2-Methyl-1,4-naphthoquinone" page 476; column 1; XP002183396 & JP 52 108959 A (NIPPON JORYU KOGYO K K) 12 September 1977 (1977-09-12)
- RICHARD T ARNOLD ET AL: "Quinones by the peroxide oxidation of aromatic compounds" JOURNAL OF ORGANIC CHEMISTRY., vol. 5, 1940, pages 250-252, XP001041459 AMERICAN CHEMICAL SOCIETY. EASTON., US ISSN: 0022-3263

## Description

### Field of the invention

The present invention relates to a process for the preparation of 2-methyl-1 ,4-naphthoquinone (vitamin K₃, "menadione"). The present invention particularly relates to a process for producing 2-methyl-1,4-naphthoquinone which is used as antihemoragic agent and in animal feed, by the oxidation of 2-methylnaphthalene with hydrogen peroxide in the presence of acetic acid.

### Background of the invention

Oxidation is one of the major and industrially important processes and widely used in the synthesis of low and high volume chemicals and pharmaceutical industry. Furthermore, the traditional route of oxidation using mineral acids and inorganic oxides suffer from the disadvantages of high capital cost, reactor corrosion, formation of by product and the difficulty in the catalyst regeneration. In recent times, scientists worldwide have been devoting their attention to the development of environmentally friendly catalysts and economically feasible routes.

Oxidation of arenes is important in chemical industries, especially in agrochemical and pharmaceutical industries. Many oxidation and hydroxylation reactions were studied using zeolites and redox molecular sieves (Sheldon et al. Curr. Opin. Solid. State Mater. Sci. 1, 1996, 101; Sheldon et al. J. Mol. Catal. A 107, 1996, 75; Kumar et al. Synlett. 1995, 289; Arends et al. Angew. Chem. Int. Ed. Engl. 36, 1997, 1145).

Oxidation of 2-Methylnaphthalene with a sulfuric acid solution of chromic acid was studied (Fieser, J. Biol. Chem., 133, 1940, 391; Sheldon, Top. Curr. Chem., 164, 1993, 21). However, in this stoichiometric oxidation about 18 kg of chromium containing waste is produced for 1 kg of product. Oxidation of 2-methylnaphthalene was investigated in presence of acetic acid with hydrogen peroxide and methyl trioxo rhenium (Adam et al., Angew. Chem. Int. Ed., 33, 1994, 2475; Herrmann et al., J. Mol. Catal. A Chemical, 138, 1999, 115). Oxidation of 2-Methylnaphthalene was carried over Pd-polystyrenesulfonic acid resin in the acetic acid with hydrogen peroxide (Yamaguchi et al., Chem. Lett., 1985, 827). Metalloporphyrin catalyzed oxidation of 2-methylnaphthalene by potassium monopersulfate was studied (Song et al., J. Org. Chem., 62, 1997, 673). The oxidation of 2-methylnaphthalene has been carried out using ammonium persulfate as an oxidizing agent in the presence of cerium (IV) ammonium sulfate and silver nitrate in an emulsified solution (Skarzewski, Tetrahedron, 40, 1984, 4997). The synthesis of vitamin K3 from different starting materials like 1-naphthol, 1,4-naphthoquinone was examined using organic oxidizing agents (Rama Rao et al. Indian J. Chem. 24 B, 1985, 233).

Oxidation of 2-methylnaphthalene using hydrogen peroxide in the presence of acid catalysts have been claimed in patents (Sugano et al. Ger. Offen DE. 2341463, 1974; Takanobu et al. Japanese kokai 77, 1977, 108959 ; Baba et al. Japanese kokai 76, 1976, 50147).

The drawbacks of using inorganic oxidants and mineral acids as given in the referred work are: (I) Catalyst can not be reused, (ii) Disposal of acid is not environmentally safe and it is not economical, (iii) Low selectivity is frequently observed, (iv) Corrosion of the reaction vessel and reactors, (v) Not easy to handle and (vi) High inventory of the catalyst.

US-A-2373003 describes the oxidation of 2-methylnaphthalene (5g) with 30% hydrogen peroxide (15cc) and glacial acetic acid (75cc) at 80°C for 10 hours to give 2-methyl-1,4-naphthoquinone in 30% yield.

### Objects of the invention

The main object of the present invention is to develop low cost and ecofriendly route for the oxidation of 2-methylnaphthalene.

Another object of the present invention is to provide a process for the preparation of 2-methyl-1,4-naphthoquinone.

### Summary of the invention

Accordingly, the present invention relates to a process for oxidation of 2-methylnaphtalene with hydrogen peroxide using 5N-17N acetic acid for 1 to 3 hours in the absence of a solid catalyst wherein the molar ratio of 2-methylnaphthalene to hydrogen peroxide is in the range of the 1:2 to 1:12 and separating the 2-methyl-1,4-naphthoquinone from the reaction mixture.

In another embodiment of the present invention, the reaction is carried out in a temperature range from 60 to 100°C.

### Detailed description of the invention

The present invention provides a process for oxidation of 2-methylnaphthalene with hydrogen peroxide in acetic acid. The reaction of the invention avoids the use of solid acid catalysts in the preparation of 2-methyl-1, 4-naphthoquinone.

### EXAMPLE 1

20 ml of acetic acid was taken in 50 ml round bottom flask and 1 g of 2-methylnaphthalene added and kept on a magnetic stirrer. The reaction mixture was heated to 100°C. After reaching 100°C hydrogen peroxide was added slowly and allowed the reaction mixture for 3 hrs at 100°C. The reaction mixture was analysed using a CHEMITO 8510 Gas Chromatography using 20 % SE-30 column coupled with flame ionization detector for product distribution.

A typical Gas Chromatography - Mass spectral fragmentation pattern and Proton Nuclear Magnetic Resonance spectra of the products, ¹H NMR proves that the product obtained was 2-methyl-1,4-naphthoquinone. The reaction was carried out using different molar ratios of 2-methylnaphthalene to hydrogen peroxide (30%) as 1:2, 1:4, 1:6, 1:8 and 1:10. Product was analyzed and the conversion and selectivity are given in Table 1.

**Table - 1 Conversion and selectivities using different molar ratios of 2-methylnaphthalene to hydrogen peroxide (30%).**

| Molar ratio of 2-methylnaphthalene to Hydrogen Peroxide | Conversion of 2-Methyl Naphthalene (%) | Selectivity of 2-Methyl-1,4-Naphthoquinone (%) |
|---|---|---|
| 1:2 | 9 | 88 |
| 1:4 | 21 | 90 |
| 1:6 | 42 | 90 |
| 1:8 | 86 | 95 |
| 1:10 | 100 | 100 |

### EXAMPLE 2

20 ml of acetic acid was taken in 50 ml round bottom flask and 1 g of 2-methylnaphthalene added and kept on a magnetic stirrer. The reaction mixture was heated to 100°C. After reaching 100°C hydrogen peroxide was added slowly and allowed the reaction mixture for 3hrs at 100°C. The reaction was carried out using 5 to 17 N concentrations of acetic acid. Product was analyzed and the conversion and selectivity are given in Table 2.

**Table - 2 Conversion and selectivities with concentration of acetic acid**

| Concentration of acetic acid (N) | Conversion of 2-methyl naphthalene (%) | Selectivity of 2-methyl-1,4-naphthoquinone (%) |
|---|---|---|
| 5 | 35 | 91 |
| 10 | 75 | 100 |
| 17 | 100 | 100 |

### EXAMPLE 3

20 ml of acetic acid was taken in 50 ml round bottom flask and I g of 2-methylnaphthalene added and kept on a magnetic stirrer. The reaction mixture was heated to 100°C. After reaching 100°C, hydrogen peroxide was added slowly and allowed the reaction mixture for 3hrs at 100°C. The reaction was carried out using 10 to 50 % concentration of hydrogen peroxide. Product was analyzed and the conversion and selectivity are given in the Table 3.

**Table - 3 Conversion and selectivities with concentration of hydrogen peroxide**

| Concentration of Hydrogen peroxide (%) | Conversion of 2-methyl.. Naphthalene (%) | Selectivity (%) 2-methyl-1,4-naphthoquinone | 6-methyl-1,4-naphthoquinone |
|---|---|---|---|
| 10 | 55 | 97 | - |
| 20 | 66 | 97 | - |
| 30 | 100 | 100 | - |
| 50 | 75 | 87 | 13 |

### EXAMPLE 4

20 ml of acetic acid was taken in 50 ml round bottom flask and 1 g of 2-methylnaphthalene added and kept on a magnetic stirrer. The reaction mixture was heated to 100°C. After reaching 100°C hydrogen peroxide was added slowly and allowed the reaction mixture for heating at 100°C. The reaction was carried out for different time periods ranging from 30 min to 240 min. Products were analyzed and the conversion and selectivity are given in the Table 4.

**Table - 4 Conversion and selectivities with the variation in the time**

| Reaction time (min) | Conversion of 2-methyl Naphthalene (%) | Selectivity (%) 2-methyl-1,4-naphthoquinone | 6-methyl-1,4-naphthoquinone |
|---|---|---|---|
| 30 | 48 | 100 | - |
| 60 | 87 | 100 | - |
| 90 | 86 | 100 | - |
| 120 | 95 | 100 | - |
| 150 | 96 | 100 | - |
| 180 | 100 | 100 | - |
| 240 | 91 | 90 | 10 |

### EXAMPLE 5

1 g of 2-methylnaphthalene taken in 50 ml round bottom flask and acetic acid was added and was kept on a magnetic stirrer. The reaction mixture was heated to 100°C. After reaching 100°C hydrogen peroxide was added slowly and allowed the reaction mixture for 3hrs at 100°C. The reaction was carried out by changing the amount of acetic acid from 5 to 20 ml. Product was analyzed and the conversion and selectivity are given in the Table 5.

**Table - 5 Conversion and selectivities changing the amount of acetic acid**

| Acetic Acid (ml) | Conversion of 2-methylnaphthalene (%) | Selectivity of 2-methyl-1,4-naphthoquinone (%) |
|---|---|---|
| 5 | 0 | 0 |
| 10 | 24 | 100 |
| 15 | 89 | 100 |
| 20 | 100 | 100 |

### EXAMPLE 6

20 ml of acetic acid was taken in 50 ml round bottom flask and I g of 2-methylnaphthatene added and kept on a magnetic stirrer and hydrogen peroxide was added slowly and allowed the reaction mixture for 3hrs while stirring. The reaction was carried out by varying the reaction temperature from 40 to 100°C. Product was analyzed and the conversion and selectivities are given in the Table 6.

**Table - 6 Conversion and selectivities with reaction temperature**

| Reaction temperature (°C) | Conversion of 2-methylnaphthalene (%) | Selectivity of 2-methyl-1,4-naphthoquinone (%) |
|---|---|---|
| 40 | 7 | 100 |
| 60 | 40 | 100 |
| 80 | 52 | 100 |
| 100 | 100 | 100 |

This method provides the following advantages compared with the conventional process (i) high conversion and selectivity are frequently observed, (ii) no waste is produced which is an ecofriendly process, (iii) do not corrode reaction vessel or reactors and (iv) it is a very economical process as there is no involvement of solid catalysts.

In view of the above, it will be seen that several advantages of the invention are achieved and other advantageous results attained.

## Claims

1. A process for the preparation of 2-methyl-1,4-naphthoquinone, said process comprising the steps of oxidising 2-methylnaphthalene with hydrogen peroxide in the presence of 5N ― 17 N acetic acid for 1 to 3 hours in the absence of a solid catalyst wherein the molar ratio of 2-methylnaphthalene to hydrogen peroxide is in the range of 1:2 to 1:12 and separating the 2-methyl-1,4-naphthoquinone so obtained.

2. A process as claimed in claim 1 wherein the temperature is in the range of 60-100°C.

## Patentansprüche

1. Verfahren zur Herstellun von 2-Methyl-1,4-naphthochinon, das die Oxidation von 2-Methylnaphthalin mit Wasserstoffperoxid in Gegenwart von 5 N - 17 N Essigsäure während 1 bis 3 Stunden in Abwesenheit eines Feststoffkatalysators, wobei das Molverhältnis von 2-Methylnaphthalin zu Wasserstoffperoxid im Bereich von 1:2 bis 1:12 liegt, und das Abtrennen des so erhaltenen 2-Methyl-1,4-naphthochinons umfaßt.

2. Verfahren nach Anspruch 1, wobei die Temperatur im Bereich von 60 bis 100°C liegt.

## Revendications

1. Procédé de préparation de 2-méthyl-1,4-naphthoquinone, ledit procédé comprenant les étapes d'oxydation du 2-méthylnaphthalène par le peroxyde d'hydrogène en présence d'acide acétique 5N-17N pendant 1 à 3 heures en absence d'un catalyseur solide, le rapport molaire du 2-méthylnaphthaléne au peroxyde d'hydrogène se trouvant dans le domaine de 1:2 à 1:12, et de séparation de la 2-méthyl-1,4-naphthoquinone ainsi obtenue.

2. Procédé tel que revendiqué dans la revendication 1, la température se trouvant dans le domaine de 60 à 100°C.
